# EUROPEAN PATENT APPLICATION

(11) **EP 4 473 827 A1**
(43) Date of publication of application: **11.12.2024**
(21) Application number: 23177615.4
(22) Date of filing: 06.06.2023
(51) Int. Cl.: A01H 1/00, A01H 5/06, A01H 6/06

(54) **CAVITY SPOT RESISTANT CARROT PLANTS**

(71) Applicant: Bejo Zaden B.V., 1749 CZ Warmenhuizen (NL)
(72) Inventor: KATSCHNIG, Diana, 1749 CZ WARMENHUIZEN (NL); STEENTJES, Rudie, Johannes, Theodorus, 1749 CZ WARMENHUIZEN (NL); HAARSMA, Adriana, Dorien, 1749 CZ WARMENHUIZEN (NL); DEKKER, Peter, Arnoldus, 1749 CZ WARMENHUIZEN (NL); SCHRIJVER, Albertus, Johannes, Maria, 1749 CZ WARMENHUIZEN (NL)
(74) Representative: Arnold & Siedsma

(57) **Abstract**

The present invention relates to cavity spot resistant carrot plants, methods for providing cavity spot resistant carrot plants and use of one or more resistance providing genomic fragments for identifying, or providing carrot plants resistant to cavity spot. Specifically, the present invention relates to carrot plants being resistant to cavity spot, wherein the carrot plants comprise one or more genomic fragments are obtainable, obtained, derived, or are from one or more carrot plants selected from the group consisting of carrot plants deposited under deposit numbers NCIMB 44133, NCIMB 44134, NCIMB 44135, and NCIMB 44136, or any one combination thereof.

## Description

The present invention relates to cavity spot resistant carrot plants, methods for providing cavity spot resistant carrot plants and use of one or more cavity spot resistance providing genomic fragments for identifying, or providing, carrot plants resistant to cavity spot.

Carrot, or *Daucus carota,* is a cultivated plant from the Umbelliferae (or Apiaceae) family, which is common in many parts of the world. The Umbelliferae family encompasses more than 3,500 species, including next to the genus *Daucus,* various other cultivated plants, for example, caraway, celery, coriander, dill, fennel, parsley, and parsnip.

Wild carrot, *Daucus carota L.,* has a white taproot that is initially edible, but becomes woody after prolonged growth. The cultivated carrot, *Daucus carota,* and especially *Daucus carota* ssp. *sativus* is a root vegetable that is usually orange, but purple, red, yellow, and white varieties are also known.

In moderate climate zones, *Daucus carota* is a biennial plant that grows vegetatively in the first year after sowing. After overwintering, the plant will flower in the second year of cultivation. In tropical and subtropical areas, the carrot plant has an annual life cycle, and the shift from vegetative to generative growth occurs without vernalization.

Two types of male sterility are described in the genus *Daucus.* In the so-called brown anther type, anthers degenerate and shrivel before they can spread pollen. In the petaloid type, the stamens are replaced by petal-like structures.

Male sterility observed in cultivated carrots is generally due to cytoplasmic male sterility caused by mitochondrial defects. Since mitochondria are transferred to the offspring by egg cells only, this trait is maternally inherited. Male sterility is a useful trait in carrot breeding as it enables 100 % cross-pollination. As a result, hybrids of *Daucus* are readily produced. Moreover, heterosis, or hybrid vigour, can be very strong in carrot.

Carrot is cultivated for its nutritious taproot. A major part of this root consists of an outer phloem cortex and an inner xylem core. Moreover, a large proportion of the cortex relative to the core is considered to have high horticultural quality. The taproot is rich in carotene, especially β-carotene, an important antioxidant that can be metabolized to vitamin A. Carrots are also a source of dietary fibre, vitamins C, B6, and K, and the antioxidant falcarinol. Antioxidants (including carotenoids) have been studied for their ability to prevent chronic disease.

The root length of carrot plants varies from 5 up to 40 cm, while the diameter can vary between 1 to 10 cm. Taproots also come in various shapes. Round, conical, or more cylindrical shapes are preferred depending on the purpose. The taproot of wild varieties is white, but cultivated carrots are ordinarily orange, sometimes red, purple, black, or yellow.

Carrots are cultivated globally. In 2011, more than 35 million tons of carrots were produced. Yet, many pests and pathogens are known that threaten harvests around the globe. These include bacterial, fungal, viral, and viroid diseases, but also insect and nematode pests. Major bacterial and fungal diseases are caused by, among others: *Xanthomonas hortorum, Erwinia carotovora, Alternaria dauci, Alternaria radicina, Pythium* spp., *Rhizoctonia* spp., *Sclerotinia* spp., *Fusarium* spp, *Botrytis cinerea,* and *Phytophthora* spp.. Nematodes, such as *Heterodera carotae, Meloidogyne* spp. and *Pratylenchus* spp., cause severe damage to the taproot, resulting in yield loss and a product unsuitable for market.

Cavity spot disease is caused by soil-borne oomycetes from the *Pythium* genus. *Pythium sulcatum* and *Pythium violae* are the two most pathogenic species within the *Pythium* genus. *Pythium* species cause diseases predominately on members of the apiaceous family, the most common being *Daucus carota. Pythium* spp. can, besides cavity spot disease, also cause damping off in the seedling stage. Seedling damping off effects the critical period after sowing. Infected seedlings rarely survive to produce a vigorous plant.

Cavity spot has been reported in most carrot producing regions of the world. The diversity of pathogenic *Pythium* spp. is thought to be broad and may vary between geographical regions in the world.

Cavity spots are watery or suberized sunken elliptical lesions at any spot along the carrot root. Infection starts as pinhead-size lesions. Cavity spot disease significantly reduces marketability of carrots resulting in carrots becoming unacceptable for the market.

Heavy rain and poorly drained soil are favorable for disease development. Metalaxyl and zoxamide can be used for chemical control, however, repeated exposure results in loss of sensitivity. Since 1980s various *Pythium* populations have been known to have resistance to metalaxyl. Additionally, this compound is increasingly banned in EU and other regions.

Due to resistance of the pathogen to fungicides, (2) limitation of allowed quantities or banning of use of fungicides in vegetable production, (3) demand for organic seed production, there is a need to introduce carrot cultivars with genetically encoded resistance to *Pythium* spp.

Because on the one hand the pathogen is becoming resistant to fungicides and the use of fungicides in vegetable production is either limited or completely banned, and on the other hand a demand from the market for organic seed, where the use of fungicides is not allowed, there is a need to introduce carrot cultivars with genetically encoded resistance to *Pythium* spp.

Considering the above, there is a thus a need in the art for providing genetically encoded resistance to cavity spot in carrots.

It is an object of the present invention, amongst other objects, to meet the above need in the art.

According to the present invention, the above need is met as outlined in the appended claims.

Specifically, the above need is met by providing carrot plants being resistant to cavity spot, wherein the plant comprises one or more genomic fragments providing resistance to cavity spot.

According to a preferred embodiment, the present one or more genomic fragments are obtainable, obtained, derived, or are from one or more carrot plants selected from the group consisting of carrot plants deposited under deposit numbers NCIMB 44133, NCIMB 44134, NCIMB 44135, and NCIMB 44136 on the 11th of April, 2023 (NCIMB Limited, Wellheads Place, Dyce, Aberdeen, AB21 7GB, United Kingdom).

According to other preferred embodiments, the present carrot plant being resistant to cavity spot comprise:
- one or more first genomic fragments providing resistance to cavity spot, wherein the one or more first genomic fragments are obtainable, obtained, derived, or are from a carrot plant deposited under deposit number NCIMB 44133; or
- one or more second genomic fragments providing resistance to cavity spot, wherein the one or more second genomic fragments are obtainable, obtained, derived, or are from a carrot plant deposited under deposit number NCIMB 44134; or
- one or more third genomic fragments providing resistance to cavity spot, wherein the one or more third genomic fragments are obtainable, obtained, derived, or are from a carrot plant deposited under deposit number NCIMB 44135; or
- one or more fourth genomic fragments providing resistance to cavity spot, wherein the one or more fourth genomic fragments are obtainable, obtained, derived, or are from a carrot plant deposited under deposit number NCIMB 44136.

According to an especially preferred embodiment, the present invention relates to carrot plant comprising any one combination of genomic fragments providing resistance to cavity spot as defined above.

According to yet another preferred embodiment, the present invention relates to carrot plant selected from the group consisting of carrot plants deposited under deposit numbers NCIMB 44133, NCIMB 44134, NCIMB 44135, and NCIMB 44136.

The present invention also relates to progeny of carrot plants as defined above.

The present carrot plants, or progeny thereof, are preferably cytoplasmic male sterile (CMS) and/or hybrids.

Advantageously, the present carrot plants, or progeny thereof, are, besides being resistant to cavity spot, further resistant to one or more, preferably all, plant pathogens selected from the group consisting of *Alternaria radicina, Alternaria dauci* and *Cercospora carotae.*

Considering the beneficial characteristics of the carrot plants described above, the present invention also relates to seeds, egg cells, callus, suspension culture, somatic embryos, clones, embryos, or plant parts of the present carrot plants.

The present invention further relates to methods for providing a carrot plant being resistant to cavity spot, wherein the method comprises the step of introgression, or introduction, of any one combination of genomic fragments providing resistance to cavity spot as defined above into a carrot plant.

The present invention furthermore relates to the use of one or more genomic fragments providing resistance to cavity spot as defined above, or of any one combination of genomic fragments providing resistance to cavity spot as defined above for identifying. or providing, a carrot plant being resistant to cavity spot.

The present invention will be further detailed in the example below.

### Example Field trial for assessing the resistance to Pythium spp

Field tests for assessing resistance against *Pythium spp.* The inoculum of *Pythium spp* and was prepared by growing the fungus in V8 nutrient medium for 7-10 days incubated at room temperature in the dark. Subsequently, agar plugs with mycelium were placed on a mixture of vermiculite with V8 broth supplemented with calcium carbonate. The mixture was grown for six weeks at room temperature in the dark. Plants were sown directly in the field in June in the Netherlands, and, after germination, thinned to a 2 cm distance between plants. As susceptible control varieties Newark F1 was used. Seven-week-old plants were inoculated by spreading the vermiculite mixture with mycelium between the plants, separate fields for *Pythium spp,* preferably during rainy weather or with additional irrigation. The inoculation was performed once or twice in the beginning of the season (June till November). The assessment of disease resistance was performed visually by the amount and density of the lesions that form on the root surface, using a classification scale from **S** (susceptible, severe symptoms) to **IR** (intermediate resistant, fewer lesions as compared to susceptible). A score on this classification scale is referred to as a disease score. The results of assessment of resistance against *Pythium spp.* summarized in **Table 1** below.

**Table 1 Disease score of different varieties for Pythium spp.**

| Cultivar | Score for *Pythium spp.* |
|---|---|
| Newark F1 (susceptible) | S |
| NCIMB 44134 | IR |
| NCIMB 44133 | IR |
| NCIMB 44136 | IR |
| NCIMB 44135 | IR |

## Claims

1. Carrot plant being resistant to cavity spot, wherein the plant comprises one or more genomic fragments providing resistance to cavity spot.

2. Carrot plant according to claim 1, wherein the one or more genomic fragments are obtainable, obtained, derived, or are from one or more carrot plants selected from the group consisting of carrot plants deposited under deposit numbers NCIMB 44133, NCIMB 44134, NCIMB 44135, and NCIMB 44136.

3. Carrot plant according to claim 1 or claim 2, wherein the carrot plant comprises one or more first genomic fragments providing resistance to cavity spot, wherein the one or more first genomic fragments are obtainable, obtained, derived, or are from a carrot plant deposited under deposit number NCIMB 44133.

4. Carrot plaint according to claim 1 or claim 2, wherein the carrot plant comprises one or more second genomic fragments providing resistance to cavity spot, wherein the one or more second genomic fragments are obtainable, obtained, derived, or are from a carrot plant deposited under deposit number NCIMB 44134.

5. Carrot plaint according to claim 1 or claim 2, wherein the carrot plant comprises one or more third genomic fragments providing resistance to cavity spot, wherein the one or more third genomic fragments are obtainable, obtained, derived, or are from a carrot plant deposited under deposit number NCIMB 44135.

6. Carrot plaint according to claim 1 or claim 2 wherein the carrot plant comprises one or more fourth genomic fragments providing resistance to cavity spot, wherein the one or more fourth genomic fragments are obtainable, obtained, derived, or are from a carrot plant deposited under deposit number NCIMB 44136.

7. Carrot plant comprising any one combination of genomic fragments providing resistance to cavity spot as defined in the preceding claims.

8. Carrot plant selected from the group consisting of carrot plants deposited under deposit numbers NCIMB 44133, NCIMB 44134, NCIMB 44135, and NCIMB 44136.

9. Progeny of a carrot plant according to any one of the claims 1 to 8.

10. Carrot plant, or progeny thereof, according to any one of the claims 1 to 9,
wherein the plant or progeny is cytoplasmic male sterile (CMS) and/or wherein the plant or progeny is a hybrid.

11. Carrot plant, or progeny thereof, according to any one of the claims 1 to 9,
wherein plant is further resistant to one or more, preferably all, plant pathogens selected from the group consisting of *Alternaria radicina, Alternaria dauci* and *Cercospora carotae.*

12. Seeds, egg cells, callus, suspension culture, somatic embryos, clones, embryos, or plant parts of a carrot plant according any one of the claims 1 to 11.

13. Method for providing a carrot plant being resistant to cavity spot, the method comprises the step of introgression, or introduction, of any one combination of genomic fragments providing resistance to cavity spot as defined in the preceding claims into a carrot plant.

14. Use of one or more genomic fragments providing resistance to cavity spot as defined in the preceding claims, or of any one combination of genomic fragments providing resistance to cavity spot as defined in the preceding claims for identifying. or providing, a carrot plant being resistant to cavity spot.
